# EUROPEAN PATENT SPECIFICATION

(11) **EP 2 902 384 B1**
(45) Date of publication and mention of the grant of the patent: **08.11.2017**
(21) Application number: 15151073.2
(22) Date of filing: 11.02.2011
(51) Int. Cl.: C07D 223/16

(54) **FORM OF IVABRADINE HYDROCHLORIDE**
FORM VON IVABRADIN-HYDROCHLORID
FORME DE CHLORHYDRATE D'IVABRADINE

(30) Priority: 12.02.2010 SI 201000057
(43) Date of publication of application: 05.08.2015
(62) Divisional of application: 11702672.4
(73) Proprietor: KRKA, D.D., Novo Mesto, 8501 Novo mesto (SI)
(72) Inventor: KOTAR-JORDAN, Berta, 8311 Kostanjevica na Krki (SI); GOJAK, Urska, 1000 Ljublijana (SI); SMRKOLJ, Matej, 1411 Izlake (SI)
(74) Representative: Andrae | Westendorp Patentanwälte Partnerschaft

(56) References cited:
- EP-A1- 1 589 005
- EP-A1- 1 695 709
- EP-A1- 1 695 710
- EP-A1- 1 695 965
- EP-A1- 1 707 562
- EP-A1- 1 775 287
- EP-A1- 1 775 288
- WO-A2-2008/146308
- CAIRA: "Crystalline Polymorphism of Organic Compounds", TOPICS IN CURRENT CHEMISTRY, SPRINGER, BERLIN, DE, vol. 198, 1 January 1998 (1998-01-01), pages 163-208, XP008166276, ISSN: 0340-1022
- "Design, Development, and Scale-up of Formulation and Process" In: "Developing Solid Oral Dosage Forms: Pharmaceutical Theory and Practice", 1 January 2009 (2009-01-01), Academic Press (imprint of Elsevier Inc), XP055372134, pages 414-415,

## Description

### FIELD OF THE INVENTION

The present invention relates to novel crystalline forms of ivabradine hydrochloride and pharmaceutical compositions prepared therefrom.

### BACKGROUND OF THE INVENTION

Ivabradine and addition salts thereof with a pharmaceutically acceptable acid have very valuable pharmacological and therapeutic properties, especially bradycardic properties, making those compounds useful in the treatment or prevention of various clinical situations of myocardial ischemia such as angina pectoris, myocardial infarct and associated rhythm disturbances, and also in various pathologies involving rhythm disturbances, especially supraventricular rhythm disturbances, and in heart failure.

The preparation and therapeutic use of ivabradine hydrochloride have been described in EP534859. Novel crystalline forms of ivabradine hydrochloride have been described in EP1589005, EP1695710, EP1695709, EP1707562, EP1695965, EP1775288 and EP1775287. Improved processes for preparation of ivabradine are disclosed in EP1589005, EP1589014, EP1614687, EP1598333, WO2008065681

WO 2008/146308 A2 encompasses a process for the preparation of highly pure ivabradine hydrochloride by treating ivabradine with alcoholic hydrogen chloride. WO 2008/146308 A2 further encompasses amorphous ivabradine hydrochloride and a process for its preparation using suitable acid addition salts of ivabradine.

### BRIEF DESCRIPTION OF THE DRAWINGS

Figure 1 is an X-ray powder diffraction pattern of ivabradine hydrochloride form Z.
Figure 2 is an X-ray powder diffraction pattern of ivabradine hydrochloride form X.
Figure 3 is an X-ray powder diffraction pattern of ivabradine hydrochloride form K.

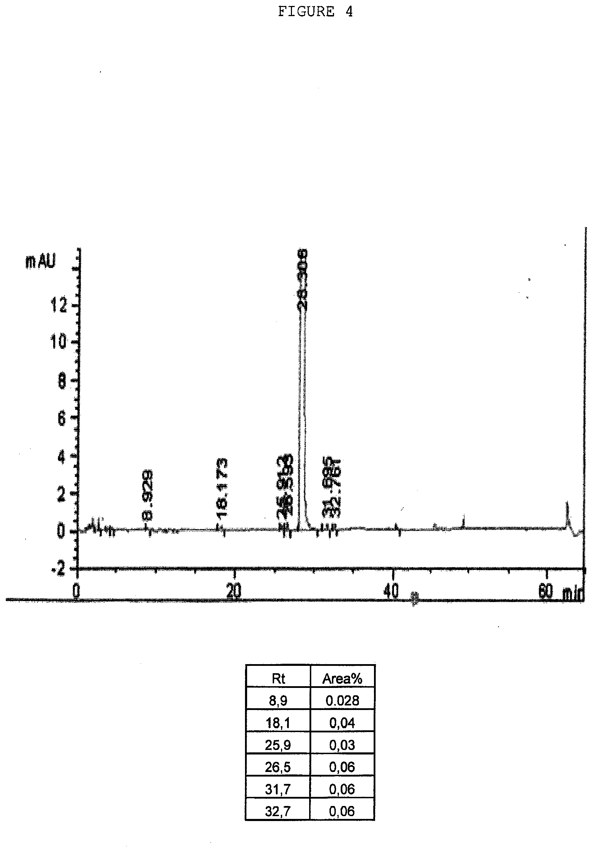

Figure 4 is a HPLC chromatogram of ivabradine in tablets prepared according to example 23 after being held at 50°C/75% RH for 1 month.

The X-ray powder diffraction pattern was obtained by Philips PW3040/60 X'Pert powder diffractometer, X'celerator detector at CuKα radiation, 1.54178 Å, 3°<2θ<30°.

HPLC chromatogram of related substances of ivabradine was obtained on a high performance liquid chromatograph with UV detector. Chromatographic separation was achieved using Atlantis dC18 150×4.6 mm, 3 µm analytical column. Chromatographic conditions used were: sample concentration of 1.5 mg/ml, detection at 286 nm, flow of 1.0 ml/min, volume of injection 5 µl. A gradient elution using mobile phase A (0.01M CH₃COONH₄ pH 7.5) and mobile phase B (CH₃CN) was employed with time table (0min 20%B, 10min 20%B, 25min 30%B, 30min 30%B, 40min 70%B, 45min 85%B, 60min 85%B, 60.5min 20%B,65min 20%B,).

### DETAILED DESCRIPTION OF THE INVENTION

A novel crystalline form of a drug substance may display different melting point, hygroscopicity, stability, solubility and/or dissolution rate, crystallinity, crystal habits, bioavailability and formulation handling characteristics, which are among the numerous properties that need to be considered in preparing a medicament that can be effectively administered.

We have found novel crystalline forms of ivabradine hydrochloride suitable as starting compounds in the preparation of final dosage forms.

According to an aspect, ivabradine hydrochloride form X, as defined in claim 1 annexed, is provided.

According to a further aspect, a process for preparation of ivabradine hydrochloride form X, as defined in claim 2 annexed, is provided.

According to a further aspect, the use of ivabradine hydrochloride for the preparation of pharmaceutical composition, as defined in claim 5 annexed, is provided.

According to a further aspect, the use of ivabradine hydrochloride form X for the preparation of ivabradine hydrochloride form K, as defined in claim 6 annexed, is provided.

According to a further aspect, ivabradine hydrochloride form X for use as a medicament, as defined in claim 7 annexed, is provided.

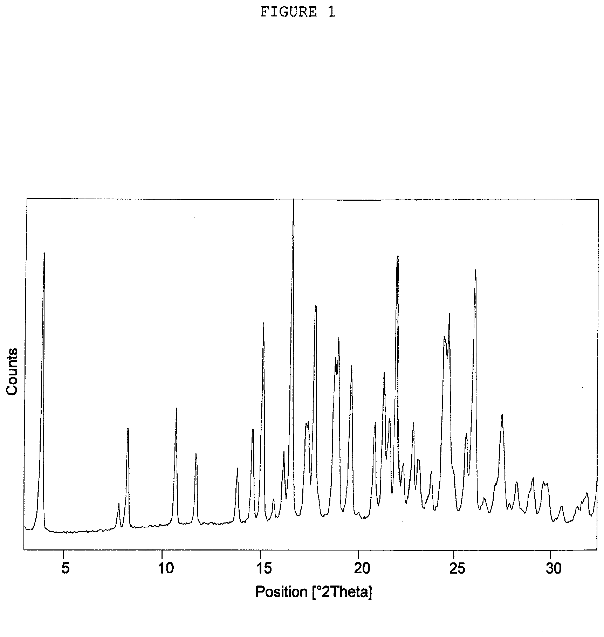

A novel crystalline form of ivabradine hydrochloride designated as form Z is characterized by an X-ray powder diffraction pattern having peaks at about 3.9, 15.1, 16.2, 16.6 and 17.8 ±0.2 degrees two-theta. Ivabradine hydrochloride form Z can be further characterized by X-ray powder diffraction peaks at about 3.9, 15.1, 16.2, 16.6, 17.8, 19.0, 22.0 and 24.7 ±0.2 degrees two-theta. The X-ray powder diffraction pattern of ivabradine hydrochloride form Z is shown in Figure 1.

A process for preparation of ivabradine hydrochloride form Z
can be characterized in that it comprises the step of:
a) dissolving amorphous ivabradine hydrochloride in isopropanol at 15-30°C, preferably at 20-25°C, more preferably at 21-23°C;
b) stirring the obtained solution at 15-30°C, preferably at 20-25°C, more preferably at 21-23°C, until ivabradine hydrochloride starts to precipitate;
c) recovering said precipitate.

Isopropanol which is brought into contact with the amorphous ivabradine hydrochloride can have a temperature of 15-30°C, preferably a temperature of 20-25°C, more preferably a temperature of 21-23°C.

The temperature of the mixture of isopropanol and amorphous ivabradine hydrochloride during the dissolving step can be a temperature of 15-30°C, preferably a temperature of 20-25°C, more preferably a temperature of 21-23°C.

The temperature of the obtained solution subjected to stirring during stirring step b) can be a temperature of 15-30°C, preferably a temperature of 20-25°_{C}, more preferably a temperature of 21-23°C.

The reaction mixture comprising the precipitate can have a temperature of 15-30°C, preferably a temperature of 20-25°C, more preferably a temperature of 21-23°C until the begin of and/or during the recovery of said precipitate.

Unless explicitly indicated otherwise, isopropanol used for dissolving compounds, such as for dissolving amorphous ivabradine hydrochloride, ivabradine and/or hydrochloric acid can generally be isopropanol of any degree of purity. According to one embodiment, isopropanol used for dissolving compounds can be pure or essentially pure isopropanol. In particular, isopropanol used for dissolving compounds can be a fluid consisting of or comprising the chemical compound isopropanol in an amount of at least 92 wt.-%, preferably in an amount of at least 97 wt.-%, more preferably in an amount of at least 99 wt.-%, each based on the total weight of said fluid consisting of or comprising isopropanol.

Ivabradine hydrochloride form Z can also be prepared by a process characterized in that it comprises the steps of:
a) dissolving ivabradine in isopropanol at 15-30°C, preferably at 20-25°C, more preferably at 21-23°C,;
b) addition of hydrochloric acid in isopropanol at 15-30°C, preferably at 20-25°C, more preferably at 21-23°C,;
c) stirring the obtained solution at 15-30°C, preferably at 20-25°C, more preferably at 21-23°C, until ivabradine hydrochloride starts to precipitate;
d) recovering said precipitate.

Isopropanol which is brought into contact with ivabradine can have a temperature of 15-30°C, preferably a temperature of 20-25°C, more preferably a temperature of 21-23°C.

Ivabradine dissolved in isopropanol can have a temperature of 15-30°C, preferably a temperature of 20-25°C, more preferably a temperature of 21-23°C, before and/or during the step of adding hydrochloric acid in isopropanol to ivabradine dissolved in isopropanol.

Both hydrochloric acid in isopropanol and ivabradine dissolved in isopropanol can have a temperature of 15-30°C, preferably a temperature of 20-25°C, more preferably a temperature of 21-23°C, before and/or during the step of adding hydrochloric acid in isopropanol to ivabradine dissolved in isopropanol.

During stirring step c) the obtained solution subjected to stirring can have a temperature of 15-30°C, preferably a temperature of 20-25°C, more preferably a temperature of 21-23°C.

The reaction mixture comprising the precipitate can have a temperature of 15-30°C, preferably a temperature of 20-25°C, more preferably a temperature of 21-23°C before the begin of and/or during the recovery of said precipitate.

The mixture hydrochloric acid in isopropanol may optionally comprise water or can be free or essentially free of water. For example, a mixture comprising hydrochloric acid, isopropanol and water may comprise HCl in an amount of at least 1 wt.-%, preferably of at least 10 wt.-%, more preferably of at least 15 wt.-%, in particular from about 20 to 38 wt.-% HCl, based on the total weight of HCl and water.

Preferably, the temperature of crystallization of ivabradine hydrochloride crystalline form Z is between 20 and 25°C, and/or the ratio of isopropanol to ivabradine hydrochloride during crystallization is preferably between 4.5 and 5.5 ml/g.

We have found that the process for preparation of crystalline ivabradine hydrochloride form Z gives ivabradine hydrochloride of a very good purity such as a purity level of more than 99.0 %, preferably 99.5 %, and most preferably 99.9 % (HPLC).

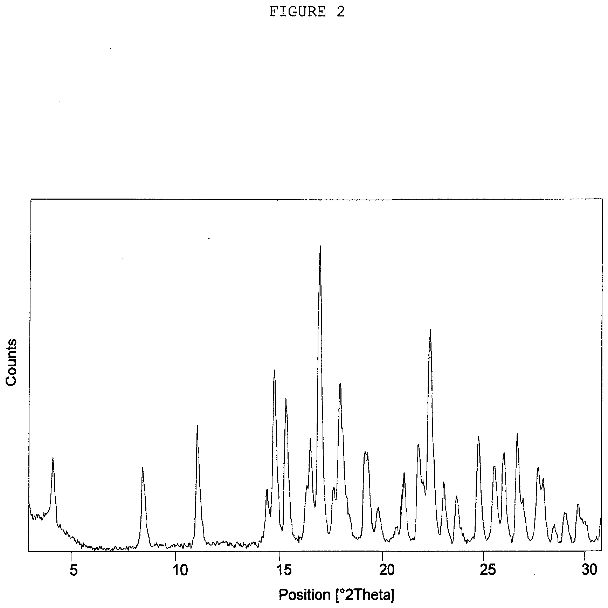

A novel crystalline form of ivabradine hydrochloride according to present invention designated as form X is characterized by an X-ray powder diffraction pattern having peaks at about 11.0, 16.5, 16.9, 21.8 and 22.4 ±0.2 degrees two-theta. Ivabradine hydrochloride form X can be further characterized by X-ray powder diffraction peaks at about 11.0, 16.5, 16.9, 21.8, 22.4, 23.7, 26.0 and 27.9 ±0.2 degrees two-theta. The X-ray powder diffraction pattern of ivabradine hydrochloride form X is shown in Figure 2.

A process for preparation of ivabradine hydrochloride form X according to present invention can be characterized in that it comprises the step of drying ivabradine hydrochloride form Z for at least 2 hours at 20-30°C.

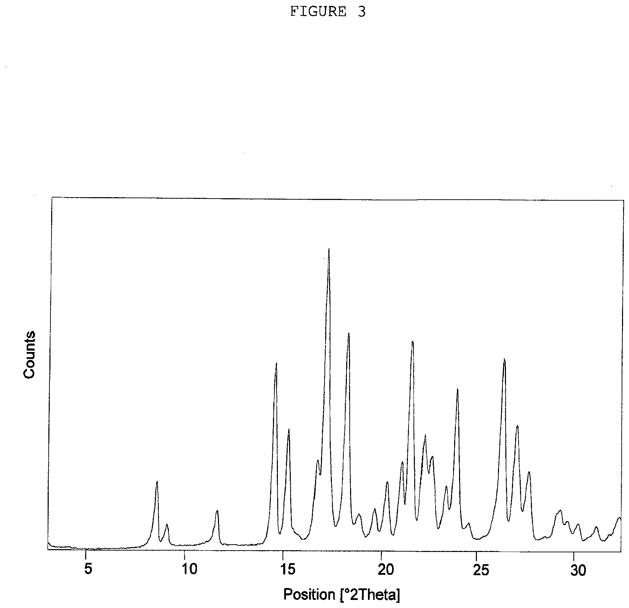

A novel crystalline form of ivabradine hydrochloride
designated as form K is characterized by an X-ray powder diffraction pattern having peaks at about 8.6, 14.6, 17.2, 18.3 and 21.6 ±0.2 degrees two-theta. Ivabradine hydrochloride form K can be further characterized by X-ray powder diffraction peaks at about 8.6, 14.6, 17.2, 18.3, 21.6, 22.3, 24.0 and 26.4 ±0.2 degrees two-theta. The X-ray powder diffraction pattern of ivabradine hydrochloride form K is shown in Figure 3.

A process for preparation of ivabradine hydrochloride form K
can be characterized in that it comprises the step of drying ivabradine hydrochloride form Z or form X for at least 2 hours at 50-80°C, preferably at 60-75°C, more preferably at 65-72°C.

The average particle size of ivabradine hydrochloride forms Z, X and K according to the present invention can be in the range of 0.1-600 µm, preferably 10-200 µm, most preferably 10-100 µm determined by laser diffraction using a Malvern Mastersizer 2000 laser diffraction instrument. The samples for analysis may be prepared in particular as set forth in the Example section.

If smaller particles are needed, the obtained ivabradine hydrochloride can be sieved, milled or micronized optionally together with other excipients. In the case of agglomerates, ultrasonication can be used.

Optical purity of ivabradine hydrochloride used in the present invention, in particular of amorphous ivabradine hydrochloride used for preparing a pharmaceutical composition, can be more than 99.0%, preferably more than 99.5%, most preferably more than 99.9% (chiral HPLC).

Ivabradine base used in the present invention can be prepared according to methods disclosed in EP534859, WO2005110993, WO2005111026, WO2005111027, WO2005123659 WO2008065681, WO2008125006, WO2008146308, WO2009062377, CN101549605, CN101343249, WO2009153461, EP2145871 and/or PCT/EP2009/009234.

The present invention also relates to pharmaceutical compositions comprising ivabradine hydrochloride, wherein ivabradine hydrochloride form X and optionally forms Z and/or K are used in the process of preparation of such compositions.

More specifically, described herein are pharmaceutical compositions comprising amorphous ivabradine hydrochloride. Active substances in an amorphous form can be better soluble and dissolve more rapidly than active substances in a crystalline form. However, it is also known that amorphous substances lack physical stability. They tend to crystallize and are hygroscopic. We have surprisingly found that amorphous ivabradine can be formulated into a stable pharmaceutical composition with improved solubility properties and improved bioavailability characteristics.

Amorphous ivabradine hydrochloride can be prepared by precipitation, in particular can be prepared by precipitation alone or on a pharmaceutically acceptable carrier. Preferably, amorphous ivabradine is prepared by spray drying the solution or dispersion comprising ivabradine hydrochloride and optionally one or more pharmaceutically acceptable carrier(s). Also, amorphous ivabradine according to present invention can be prepared by spraying the solution or dispersion of ivabradine hydrochloride on a solid carrier.

Preferably crystalline forms Z, X and K are used in the preparation of amorphous ivabradine hydrochloride, however, other crystalline forms of ivabradine hydrochloride, already know in prior art, can also be used, such as crystalline forms α, β, dβ, γ, γd, δ and δd.

For the purposes of this invention "pharmaceutically acceptable carrier" means any pharmaceutically acceptable excipient. Preferably carriers are selected from a group of stabilizers described below.

Disclosed is a process for the preparation of amorphous ivabradine hydrochloride, which process comprises:
a) providing ivabradine hydrochloride comprising or consisting of ivabradine hydrochloride selected from the group consisting of ivabradine hydrochloride crystalline forms Z, X, K, α, β, dβ, γ, γd, δ, δd, and mixtures thereof, preferably selected from the group consisting of ivabradine hydrochloride crystalline forms Z, X, K, and mixtures thereof,
b) preparing amorphous ivabradine hydrochloride from said ivabradine hydrochloride provided in step a).

Disclosed is a pharmaceutical composition comprising amorphous ivabradine hydrochloride obtained and/or obtainable according to procedures taught herein.

The pharmaceutical composition comprising amorphous ivabradine hydrochloride, in particular in the form of a tablet, can comprise at least one further ingredient, preferably at least two further ingredients, more preferably at least three further ingredients, in particular four ingredients selected from the group consisting of microcrystalline cellulose, maize starch, magnesium stearate, and colloidal silicon dioxide (in particular colloidal silicon dioxide, anhydrous).

Stabilizers can be added in granulating liquid in order to stabilize dissolved active ingredient, to increase the solubility of the drug and/or to inhibit the recrystallisation of the drug. In addition, stabilizers can be included directly to the tabletting mixture.

The term "granulating liquid" according to the present invention can refer to water, alcohol, water/alcohol mixture, or an aqueous, alcoholic or aqueous/alcoholic solution, dispersion or suspension, which contains purified water or demineralised water or lower alcohols such as methanol, ethanol, isopropanol or mixtures thereof as diluents and the substance which is dispersed, suspended or dissolved in the diluent. The said substance can have known functions of excipients used in the solid pharmaceutical composition according to the invention such as binder, surfactant, stabilizing agent or crystallization inhibitor.

There is no limitation to solvents used in the preparation of amorphous ivabradine hydrochloride, preferably the solvents are water and/or alcohols such as ethanol, methanol and/or isopropanol.

The term "coprecipitate" as used herein refers to compositions comprising amorphous ivabradine together with at least one pharmaceutically acceptable carrier, being prepared by removing solvent from a solution comprising both of them.

Pharmaceutical excipient(s) can be selected from the group consisting of fillers, diluents, binders, lubricants, disintegrants, glidants, stabilizers and film-forming agents.

One or more different fillers/diluents can be used, such as lactose (anhydrous, monohydrate, spray dried lactose etc.), microcrystalline cellulose, powdered cellulose, silicified microcrystalline cellulose, starches, pregelatinized starch, mannitol, sorbitol, lactitol, xylitol, calcium phosphate, calcium hydrogen phosphate, calcium carbonate, magnesium carbonate, sucrose, glucose, fructose, dextrates, maltodextrins, calcium lactate or combined diluents, such as Cellactose (spray-dryed lactose and powdered cellulose) and Starlac (co-dryed lactose and starch).

One or more binders can be selected frommaltodextrin, povidone or polyvinylpyrrolidone, copovidone (Plasdone S-630), microcrystalline cellulose, hydroxypropylcellulose, low-substituted hydroxypropylcellulose, hydroxyethylcellulose, hydroxypropylmethylcellulose and/or other cellulose ethers, dextrates, polyethylene glycol, starch, pregelatinized starch, gelatine, polymethacrylates.

Further, one or more disintegrants and/or superdisintegrants can also be included into a pharmaceutical composition, such as starch(es) (e.g. maize starch, potato starch), modified starch(es) (e.g. sodium starch glycolate), modified cellulose (e.g. croscarmellose, i.e. cross-linked carboxymethlycellulose sodium), cross-linked polyvinylpyrrolidone (crospovidone), low-substituted hydroxypropylcellulose, microcrystalline cellulose, carboxymethylcellulose sodium, carboxymethylstarch sodium, Amberlite®, alginic acid, sodium alginate, guar gum, gellan gum, xantan gum and/or calcium silicate.

One or more stabilizers, which improve the physical and chemical stability of amorphous ivabradine hydrochloride, can be used in a pharmaceutical composition of the present invention. Such stabilizers can be (but are not limited to) polyvinylpyrrolidone and derivatives thereof, maltodextrin, cyclodextrins (hydroxypropyl-β-cyclodextrin), xanthan gum, pectins, alginates, tragacanth, arabic gum, carrageenans, agar, hydroxypropylcellulose, bentonite, magnesium aluminium silicate, sodium hydroxide, meglumine, sodium hydrogen carbonate, calcium carbonate, calcium chloride, magnesium oxide, magnesium hydroxide, sodium hydrogen phosphate, sodium dihydrogen phosphate, potassium acetate, sodium tartrate, antioxidants such as butylated hydroxytoluene, butylated hydroxyanisole, citric acid or any other stabilizer.

Further, the pharmaceutical composition of the present invention may contain one or more lubricants. Suitable lubricants can be stearic acid, magnesium stearate, calcium stearate, sodium or magnesium laurylsulphate, hydrogenated vegetable oils and fats, hydrogenated castor oil, sodium stearyl fumarate, talc, macrogols, palmitic acid, and combinations thereof. A preferred lubricant is magnesium stearate.

One or more glidants can also be added to the composition according to the invention. They can be selected from the group comprising of talc, silicon dioxide of different grades (such as colloidal or precipitated silica) etc.

Individual excipents may have multiple functions, i.e. one excipient may function as diluent and additionally binder, binder and disintegrant etc.

Optionally, cores/tablets can be coated with one or more conventional materials used for film coating which can be used to modify the appearance of the solid composition such as smoothness of the surface, taste masking, colour or to change the physical properties of the cores such as ability to absorb moisture or interact with oxygen.

The polymers used in film coating can be either cellulose derivatives, such as the cellulose ethers (e.g. hydroxyethylcellulose, hydroxypropylcellulose, hydroxypropylmethylcellulose and methylcellulose), or acrylic polymers and copolymers. In case of functional film coating, such functional film coating in the present invention preferably has decreased permeability for gasses such as moisture and/or oxygen. Functional polymers such as polyvinyl alcohol, calcium carboxymethyl cellulose, graft copolymer of polyethylene glycol and polyvinyl alcohol obtainable as Kollicoat IR or Kollicoat Protect from company BASF, aminoalkyl methacrylate copolymer sold as Eudragit E, Eudragit EPO can be selected for obtaining such functional coating. Other excipients selected from pigment and colorant, plasticizers, glidants and/or antitacking agents can optionally be used in such functional coating.

The commonly used plasticizers are polyols (glycerol, propylene glycol, polyethylene glycols), organic esters (phthalate esters, dibutyl sebacetate, citrate esters, triacetin) and oils/glycerides (castor oil, acetylated monoglycerides, fractionated coconut oil).

Colourants/opacifiers can be added. They are classified into several groups: organic dyes and their lakes, inorganic colours, natural colours.

Layering or film coating dispersion can be prepared by using one or more different solvents (water, alcohols, ketones, esters, chlorinated hydrocarbons), preferably water.

Film coating dispersions can also be used as ready-to-make preparations which are available on the market.

The pharmaceutical composition can be prepared as any pharmaceutical solid dosage form, such as powders, granules, pellets, tablets, coated tablets, orodispersible tablets, chewable tablets, mini tablets, multi layered tablets, capsules.

The pharmaceutical formulations may be processed by direct compression, dry granulation or wet granulation, extrusion followed by spheronization, etc.

Equipment suitable for processing the pharmaceutical formulations can include blenders, granulators (high sheer, fluid bed), roller compacters, drying machines, tableting machines, coating pans, etc.

The pharmaceutical composition of ivabradine hydrochloride might be processed and packed under a modified atmosphere. The atmosphere with the modified oxygen content or reduced oxygen partial pressure may be obtained by the use of a reduced pressure atmosphere, e.g. by creating a partial vacuum by means of a suitable pump or by partial freezing or liquefying the atmosphere, or by the use of an inert gas, such as nitrogen or argon with the oxygen content of less than 8 vol.-%, preferably less than 4 viol.-%, most preferably less than 2 vol.-%, each based on the total gas volume of the respective inert gas, or by the use of oxygen absorbents, even though the high efficacy of such absorbents leading to very low oxygen levels is normally not necessary.

The composition is present in a packaging, with a blister packaging or a container made of polymeric material such as high density polyethylene or glass being preferred. Optionally, the packaging can be provided with means for trapping and disposal of free oxygen. The composition might be enclosed in a substantially gas exchange nonpermeable material as packaging which has an atmosphere with the required reduced oxygen content. The substantially gas exchange non-permeable packaging is preferably selected from the group consisting of an Al/Al blister package, an Al-polychloro-3-fluoroethylene homopolymer/PVC laminate blister or a container. Low moisture permeable primary packaging materials such as polychloro-3-fluoroethylene homopolymer/PVC laminate can be used with the thickness in the range 270 µm to 360 µm. In case of Al/Al blisters can be used laminate with the thickness in the range 100 µm to 155 µm. In a special example the solid composition in form of tablets can be packed into containers with a desiccant and/or oxygen absorber mounted into the container closure system and/or into the container walls or added as a capsule into the container filled with solid composition.

Preferred pharmaceutical compositions according to present invention are compositions comprising amorphous ivabradine hydrochloride and a stabilizer selected from maltodextrin, cyclodextrin, hydroxypropylcellulose, carrageenan and/or meglumine. More preferred are pharmaceutical compositions comprising amorphous ivabradine and a stabilizer selected from maltodextrin and/or cyclodextrin. Most preferred are pharmaceutical compositions comprising a coprecipitate of amorphous ivabradine hydrochloride and maltodextrin.

The invention is illustrated by reference to the following examples. However, the examples are not intended to limit any scope of the claim anyway.

### EXAMPLES

### Example 1: Preparation of ivabradine hydrochloride form Z

10 g of amorphous ivabradine hydrochloride is dissolved in 50 ml of isopropanol, stirred to crystallize and aged for 2 hours at 22°C. The product is filtered. 8.4 g of crystalline ivabradine hydrochloride is obtained. (KF 1.9%, LOD 29.1%)

Particle size distribution:

| average particle size [µm] | 207 |
|---|---|
| d10 [µm] | 4,2 |
| d50 [µm) | 73,5 |
| d90 [µm] | 614,8 |

The size distribution of ivabradine hydrochloride particles was determined by laser diffraction using a Malvern Mastersizer 2000 laser diffraction instrument. The samples for analysis were prepared by dispersing a weighed amount of ivabradine hydrochloride particles in isopar.

### Example 2: Preparation of ivabradine hydrochloride form Z

7.0 g ivabradine as base is dissolved in 20 ml of isopropanol and solution of 1.22 ml of konc. hydrochloric acid in 20 ml of isopropanol is gradually added. The solution is stirred to crystallize and aged for 2 hours at 22°C. The product is filtered. 8.0 g of crystalline ivabradine hydrochloride is obtained. (KF 4.2 %, LOD 17.9 % )

Particle size distribution:

| average particle size [µm] | 100 |
|---|---|
| d10 [µm] | 2,6 |
| d50 [µm) | 6,9 |
| d90 [µm] | 248,0 |

### Example 3: Preparation of ivabradine hydrochloride form X

8.5 g of ivabradine hydrochloride form Z is dried in vacuo at 22°C for 2 hours. 5.0 g of crystalline ivabradine hydrochloride is obtained. (KF 1.9 %, LOD 1.8 %)

### Example 4: Preparation of ivabradine hydrochloride form K

5.0 g of ivabradine hydrochloride form X is dried for 2 hours at 70°C. 5.0 g of crystalline ivabradine hydrochloride is obtained. (KF 0.9 %)

### Examples 5,6:Preparation of amorphous ivabradine hydrochloride

10 g of ivabradine hydrochloride is dissolved in 200 g of purified water and sprayed into spray dryer to obtain amorphous form of ivabradine hydrochloride.

10 g of ivabradine hydrochloride is dissolved in 200 g of absolute ethanol and sprayed into spray dryer to obtain amorphous form of ivabradine hydrochloride.

### Examples 7-11: Preparation of amorphous ivabradine hydrochloride coprecipitate

| Ingredients [g] | Ex. 7 | Ex. 8 | Ex. 9 | Ex.10 | Ex. 11 |
|---|---|---|---|---|---|
| Ivabradine hydrochloride | 10 | 10 | 10 | 10 | 10 |
| Polyvinylpyrrolidone (PVP K30) | 10 | / | / | / | / |
| Maltodextrin | / | 20 | / | / | / |
| Cyclodextrin (HP-β-cyclodextrin) | / | / | 30 | / | / |
| Hydroxypropylcellulose | / | / | / | 5 | / |
| Carrageenan | / | / | / | / | 0.5 |

The coprecipitate is prepared by spray drying of aqueous solution of ivabradine hydrochloride and other excipients.

### Examples 12,13: Preparation of amorphous ivabradine hydrochloride coprecipitate

| Ingredients [g] | Example 12 | Example 13 |
|---|---|---|
| Ivabradine hydrochloride | 10 | 10 |
| Sodium hydroxide | 0.8 | / |
| Meglumine | / | 3.9 |

Ivabradine hydrochloride and stabilizer are dispersed in a suitable solvent, preferably water, ethanol or their mixture thereof, and sprayed into spray dryer to obtain amorphous ivabradine coprecipitate.

### Examples 14-17: Preparation of tablets each containing 7.5 mg of ivabradine hydrochloride in a form of coprecipitate

| Ingredients [mg] | Ex. 14 | Ex. 15 | Ex. 16 | Ex. 17 |
|---|---|---|---|---|
| Example 7 | 16.170 | / | / | / |
| Example 8 | / | 24.255 | / | / |
| Example 9 | / | / | 32.340 | / |
| Example 13 | / | / | / | 11.238 |
| Microcrystalline cellulose | 106.580 | 96.495 | 88.410 | 111.512 |
| Maize starch | 25.000 | 27.000 | 27.000 | 25.000 |
| Magnesium stearate | 1.500 | 1.50 | 1.50 | 1.50 |
| Colloidal silicon dioxide, anhydrous | 0.750 | 0.75 | 0.75 | 0.75 |

Amorphous ivabradine hydrochloride coprecipitate and all other components are mixed in an appropriate mixer and pressed into 150 mg round tablets. Optionally, the tablets are film coated.

### Example 18: Preparation of tablets with amorphous ivabradine hydrochloride

| Ingredients | Amount [mg] | Amount by weight [%] |
|---|---|---|
| Ivabradine hydrochloride | 8.085* | 5.39 |
| Povidone (PVP K30) | 8.085 | 5.39 |
| Microcrystalline cellulose | 104.580 | 69.72 |
| Maize starch | 27.000 | 18.00 |
| Magnesium stearate | 1.500 | 1.00 |
| Colloidal silicon dioxide | 0.750 | 0.50 |
| Total | 150 | 100 |

| | | |
|---|---|---|
| *corresponding to 7.5 mg of ivabradine | | |

Ivabradine hydrochloride is dissolved in a pre-prepared aqueous solution of povidone to form granulating liquid. The granulating liquid is sprayed onto a mixture of maize starch, a part of microcrystalline cellulose and a part of colloidal silicon dioxide, anhydrous in a fluid-bed granulator or high shear mixer. The obtained granulate is dried and sieved. A granulate is mixed with other excipients and pressed into tablets. Optionally, the tablets are film coated with aqueous dispersion.

| Film-coating | Amount/tablet [mg] |
|---|---|
| Hypromellose 6cp | 3.000 |
| Polyethylene glycol 6000 | 0.480 |
| Glycerol | 0.195 |
| Titanium dioxide | 0.675 |
| Iron oxide, yellow | 0.090 |
| Iron oxide, red | 0.030 |
| Magnesium stearate | 0.030 |
| Total | 4.5 |

### Example 19: Preparation of tablets with amorphous ivabradine hydrochloride

| Ingredients | Amount [mg] | Amount by weight [%] |
|---|---|---|
| Ivabradine hydrochloride | 8.085* | 5.39* |
| Maltodextrin | 15.000 | 10.00 |
| Microcrystalline cellulose | 97.665 | 65.11 |
| Maize starch | 27.000 | 18.00 |
| Magnesium stearate | 1.500 | 1.00 |
| Colloidal silicon dioxide, anhydrous | 0.750 | 0.50 |
| Total | 150 | 100 |

| | | |
|---|---|---|
| *corresponding to 7.5 mg of ivabradine | | |

Ivabradine hydrochloride and matodextrin are dissolved in purified water while stirring and heating to 40°C. The obtained granulating liquid is sprayed onto a mixture of maize starch, a part of microcrystalline cellulose and a part of colloidal silicon dioxide, anhydrous in a fluid-bed granulator or high shear mixer. The obtained granulate is dried, sieved and mixed with the other part of microcrystalline cellulose and colloidal silicon dioxide, anhydrous. At the end magnesium stearate is admixed. The tabletting mixture is pressed into tablets. Optionally, the tablets are film coated with aqueous dispersion of Opadry in a suitable coating pan.

### Example 20: Preparation of tablets of amorphous ivabradine hydrochloride

| Ingredients | Amount [mg] | Amount by weight [%] |
|---|---|---|
| Ivabradine hydrochloride | 8.085* | 5.39* |
| Meglumine | 3.750 | 2.50 |
| Butylated hydroxytoluene | 0.030 | 0.02 |
| Microcrystalline cellulose (Avicel PH 101) | 55.885 | 37.26 |
| Maize starch | 30.000 | 20.00 |
| Microcrystalline cellulose (Avicel PH 200) | 50.00 | 33.33 |
| Magnesium stearate | 1.500 | 1.00 |
| Colloidal silicon dioxide, anhydrous | 0.750 | 0.50 |
| Total | 150 | 100 |

| | | |
|---|---|---|
| *corresponding to 7.5 mg of ivabradine | | |

Meglumine and butylated hydroxytoluene are dissolved in a mixture of ethanol and purified water. A weight ratio of ethanol:purified water 5:2 was used. Ivabradine hydrochloride is added and dissolved. The obtained granulating liquid is sprayed onto a mixture of Avicel PH 101 and a part of colloidal silicon dioxide, anhydrous in a suitable granulator. Ethanol and purified water evaporate during the granulating process. The dry granulate is sieved. A granulate is mixed with other excipients and pressed into tablets. Optionally, the tablets are film coated in a suitable coating pan.

### Example 21: Preparation of tablets with amorphous ivabradine hydrochloride

| Ingredients | Amount [mg] | Amount by weight [%] |
|---|---|---|
| Ivabradine hydrochloride | 8.085* | 5.39* |
| Cyclodextrin(HP-β-CD) | 36.34 | 24.23 |
| Microcrystalline cellulose (Avicel PH 101) | 61.325 | 40.88 |
| Maize starch | 27.000 | 18.00 |
| Microcrystalline cellulose (Avicel PH 200) | 15.00 | 10.00 |
| Magnesium stearate | 1.500 | 1.00 |
| Colloidal silicon dioxide, anhydrous | 0.750 | 0.50 |
| Total | 150 | 100 |

| | | |
|---|---|---|
| *corresponding to 7.5 mg of ivabradine | | |

Ivabradine hydrochloride and HP-β-CD are dissolved in purified water and sprayed onto Avicel PH 101 in a fluid-bed granulator or a high sheer mixer. Wet granulate is dried and sieved. Excess HP-β (weight ratio 2:9) is employed to ensure the complete complexation of ivabradine in solid form.

In addition, the obtained dry granulate is mixed with other excipients and pressed into tablets.

### Example 22: Preparation of tablets with amorphous ivabradine hydrochloride

| Ingredients | Amount [mg] | Amount by weight [%] |
|---|---|---|
| Ivabradine hydrochloride | 8.085* | 5.39* |
| Maltodextrin (Lycatab DSH) | 15.000 | 10.00 |
| Maize starch | 27.000 | 18.00 |
| Microcrystalline cellulose (Avicel PH101) | 47.665 | 31.78 |
| Microcrystalline cellulose (Avicel PH200) | 50.000 | 33.33 |
| Magnesium stearate | 1.500 | 1.00 |
| Colloidal silicon dioxide, anhydrous | 0.750 | 0.50 |
| Total | 150 | 100 |

| | | |
|---|---|---|
| *corresponding to 7.5 mg of ivabradine | | |

Maltodextrin (Lycatab DSH) is dissolved in purified water. Ivabradine hydrochloride is added and dissolved while heating up to 40°C. The obtained solution is sprayed onto a mixture of microcrystalline cellulose (Avicel PH101), maize starch and a part of colloidal silicon dioxide, anhydrous in a high shear mixer. The wet granulate is dried and sieved. Microcrystalline cellulose (Avicel PH200) and the other part of colloidal silicon dioxide, anhydrous are added and mixed in a suitable mixer. At the end magnesium stearate is admixed and the obtained tabletting mixture is pressed into tablets. The tablet cores are film coated with aqueous dispersion of Opadry Orange 03H32599 in a suitable coating pan. Opadry Orange 03H32599 is a ready-to-use dry blend containing polymer, plasticizer and pigment. During film coating process the weight gain of the tablet cores of 5 % is achieved.

### Example 23: Packaging of the tablets prepared as described in Example 22

The tablets of ivabradine hydrochloride from Example 22 are packed in Al/Alblisters under a nitrogen atmosphere with the oxygen content from 1 to 2 vol. %.

Stability of ivabradine hydrochloride formulation according to example 23 in comparison to amorphous ivabradine hydrochloride according to example 5, held at 50°C, 75% relative humidity:

**Table 1: Comparative stability data of amorphous ivabradine hydrochloride according to example 5 and example 23 held at 50°C and 75% relative humidity for 1 month:**

| | Rt (min) | 3.7 | 8.9 | 18.1 | 25.9 | 26.5 | 31.7 | 32.7 | total impurities (% area) |
|---|---|---|---|---|---|---|---|---|---|
| Example 23 | t0 | | | | | 0.03 | 0.05 | | 0.08 |
| | 1 month | | 0.03 | 0.04 | 0.03 | 0.06 | 0.06 | 0.06 | 0.28 |
| Example 5 | t0 | | | | | 0.03 | 0.05 | | 0.08 |
| | 1 month | 0.28 | 0.21 | 4.2 | 3.3. | 3.2 | 0.11 | 0.28 | 11.6 |

### Example 24: Preparation of tablets with amorphous ivabradine hydrochloride

| Ingredients | Amount [mg] | Amount by weight [%] |
|---|---|---|
| Ivabradine hydrochloride | 8.085* | 5.39 |
| Maltodextrin (Glucidex 19) | 18.000 | 12.00 |
| Hypromellose (Pharmacoat 603) | 1.600 | 1.07 |
| Maize starch | 27.000 | 18.00 |
| Microcrystalline cellulose (Avicel PH101) | 43.065 | 28.71 |
| Microcrystalline cellulose (Avicel PH200) | 50.000 | 33.33 |
| Magnesium stearate | 1.500 | 1.00 |
| Colloidal silicon dioxide, anhydrous | 0.750 | 0.50 |
| Total | 150 | 100 |

| | | |
|---|---|---|
| *corresponding to 7.5 mg of ivabradine | | |

Hypromellose (Pharmacoat 603) and Maltodextrin (Glucidex 19) are dissolved in heated purified water (up to 40°C). Ivabradine hydrochloride and ethanol are added and mixed until complete active substance is dissolved into clear solution. A weight ratio purified water : ethanol of 3.5 : 1 is used. The obtained granulating liquid is sprayed onto a mixture of microcrystalline cellulose (Avicel PH101) and maize starch in a fluid-bed granulator or high shear mixer. The wet granulate is dried up to the product temperature 25-60°C. The dry granulate is sieved. Microcrystalline cellulose (Avicel PH200) and colloidal silicon dioxide, anhydrous are added and mixed in a suitable mixer. At the end magnesium stearate is admixed and the obtained tabletting mixture is pressed into tablets. The tablet cores are film coated with aqueous dispersion of Opadry Orange 03H32599 in a suitable coating pan. Opadry Orange 03H32599 is a ready-to-use dry blend containing polymer, plasticizer and pigment. During film coating process the weight gain of the tablet cores of 3-5 % is achieved.

### Example 25: Preparation of tablets with amorphous ivabradine hydrochloride

| Ingredients | Amount [mg] | Amount by weight [%] |
|---|---|---|
| Ivabradine hydrochloride | 8.085* | 5.39 |
| Maltodextrin (Glucidex 19) | 18.000 | 12.00 |
| Hypromellose (Pharmacoat 603) | 1.500 | 1.00 |
| Maize starch | 27.000 | 18.00 |
| Microcrystalline cellulose (Avicel PH101) | 43.665 | 29.11 |
| Microcrystalline cellulose (Avicel PH200) | 49.500 | 33.00 |
| Magnesium stearate | 1.500 | 1.00 |
| Colloidal silicon dioxide, anhydrous | 0.750 | 0.50 |
| Total | 150 | 100 |

| | | |
|---|---|---|
| *corresponding to 7.5 mg of ivabradine | | |

Hypromellose and Maltodextrin are dissolved in heated purified water (up to 40°C). Ivabradine hydrochloride is added and dissolved. Ethanol is added into a solution. The obtained granulating liquid is sprayed onto a mixture of microcrystalline cellulose (Avicel PH101) and maize starch in a fluid-bed granulator. The dry granulate is sieved. Microcrystalline cellulose (Avicel PH200) and colloidal silicon dioxide, anhydrous are added and mixed in a suitable mixer. At the end magnesium stearate is admixed and the obtained tabletting mixture is pressed into tablets. The tablet cores are film coated with aqueous dispersion of Opadry Orange 03H32599. During film coating process the weight gain of the tablet cores of 3-5% is achieved.

### Example 26: Preparation of tablets with amorphous ivabradine hydrochloride

| Ingredients | Amount [mg] | Amount by weight [%] |
|---|---|---|
| Ivabradine hydrochloride | 8.085* | 5.39 |
| Maltodextrin (Glucidex 19) | 15.000 | 10.00 |
| Hypromellose (Pharmacoat 603) | 2.200 | 0.80 |
| Maize starch | 27.000 | 18.00 |
| Microcrystalline cellulose (Vivapur 101) | 46.965 | 31.31 |
| Microcrystalline cellulose (Vivapur 12) | 49.500 | 33.00 |
| Magnesium stearate | 1.500 | 1.00 |
| Colloidal silicon dioxide, anhydrous | 0.750 | 0.50 |
| Total | 150 | 100 |

| | | |
|---|---|---|
| *corresponding to 7.5 mg of ivabradine | | |

The formulation is prepared by wet granulation. A mixture of microcrystalline cellulose (Vivapur 101) and maize starch is granulated with an ethanol-water solution of ivabradine hydrochloride, hypromellose (Pharmacoat 603) and maltodextrin (Glucidex 19). The granulation liquid was prepared in such a way that hypromellose and maltodextrin are dissolved in heated purified water (up to 40°C). Ivabradine hydrochloride and ethanol are added and mixed until a clear solution is obtained. A weight ratio purified water : ethanol of 4 : 1 or 5 : 2 is used. The wet granulate is dried up to the product temperature 25-60°C. Prepared granulate is mixed with microcrystalline cellulose (Vivapur 12) and colloidal silicon dioxide, anhydrous. Magnesium stearate is admixed and the obtained tabletting mixture is pressed into tablets. The tablet cores are film coated with aqueous dispersion of Opadry Orange 03H32599. During film coating process the weight gain of the tablet cores of 3% is achieved.

### Example 27: Preparation of tablets with amorphous ivabradine hydrochloride

| Ingredients | Amount [mg] | Amount by weight [%] |
|---|---|---|
| Ivabradine hydrochloride | 8.085* | 5.39 |
| Maltodextrin (Glucidex 19) | 18.000 | 12.00 |
| Hypromellose (Pharmacoat 603) | 1.800 | 1.20 |
| Maize starch | 27.000 | 18.00 |
| Microcrystalline cellulose (Vivapur 101) | 43.365 | 28.91 |
| Microcrystalline cellulose (Vivapur 12) | 49.500 | 33.00 |
| Magnesium stearate | 1.500 | 1.00 |
| Colloidal silicon dioxide, anhydrous | 0.750 | 0.50 |
| Total | 150 | 100 |

| | | |
|---|---|---|
| *corresponding to 7.5 mg of ivabradine | | |

The formulation is prepared by wet granulation in a high shear mixer. A mixture of microcrystalline cellulose (Vivapur 101) and maize starch is granulated with an ethanol-water solution of ivabradine hydrochloride, hypromellose (Pharmacoat 603) and maltodextrin (Glucidex 19). Prepared granulate is mixed with microcrystalline cellulose (Vivapur 12) and colloidal silicon dioxide, anhydrous. Magnesium stearate is admixed and the obtained tabletting mixture is pressed into tablets. The tablet cores are film coated with aqueous dispersion of Opadry Orange 03H32599. During film coating process the weight gain of the tablet cores of 5% is achieved.

The tablets of amorphous ivabradine hydrochloride from Examples 24-27 are packed in Al/Alblisters.

In particular, the following items are provided herein:
1. Ivabradine hydrochloride form Z characterized by an X-ray powder diffraction pattern having peaks at about 3.9, 15.1, 16.2, 16.6 and 17.8 ±0.2 degrees two-theta.
2. Ivabradine hydrochloride according to item 1, which is further characterized by an X-ray powder diffraction pattern having peaks at about 3.9, 15.1, 16.2, 16.6, 17.8, 19.0, 22.0 and 24.7 ±0.2 degrees two-theta.
3. A process for preparation of ivabradine hydrochloride form Z according to items 1 or 2, which is characterized in that it comprises the steps of:
   a) dissolving amorphous ivabradine hydrochloride in isopropanol at 15-30°C;
   b) stirring the obtained solution at 15-30°C until ivabradine hydrochloride starts to precipitate;
   c) recovering said precipitate.
4. A process for preparation of ivabradine hydrochloride form Z according to items 1 or 2, which is characterized in that it comprises the steps of:
   a) dissolving ivabradine in isopropanol at 15-30°C;
   b) addition of hydrochloric acid in isopropanol at 15-30°C;
   c) stirring the obtained solution at 15-30°C until ivabradine hydrochloride starts to precipitate;
   d) recovering said precipitate.
5. Ivabradine hydrochloride form X characterized by an X-ray powder diffraction pattern having peaks at about 11.0, 16.5, 16.9, 21.8 and 22.4 ±0.2 degrees two-theta.
6. Ivabradine hydrochloride according to item 5, which is further characterized by an X-ray powder diffraction pattern having peaks at about 11.0, 16.5, 16.9, 21.8, 22.4, 23.7, 26.0 and 27.9 ±0.2 degrees two-theta.
7. A process for preparation of ivabradine hydrochloride form X according to items 5 or 6, which is characterized in that it comprises the step of drying ivabradine hydrochloride form Z for at least 2 hours at 20-30°C.
8. Ivabradine hydrochloride form K characterized by an X-ray powder diffraction pattern having peaks at about 8.6, 14.6, 17.2, 18.3 and 21.6 ±0.2 degrees two-theta.
9. Ivabradine hydrochloride according to item 8, which is further characterized by an X-ray powder diffraction pattern having peaks at about 8.6, 14.6, 17.2, 18.3, 21.6, 22.3, 24.0 and 26.4 ±0.2 degrees two-theta.
10. A process for preparation of ivabradine hydrochloride form K according to items 8 or 9, which is characterized in that it comprises the step of drying ivabradine hydrochloride form Z or form X for at least 2 hours at 50-80°C.
11. Use of ivabradine hydrochloride according to any one of the preceding items for the preparation of pharmaceutical composition.

## Claims

1. Ivabradine hydrochloride form X, which is **characterized by** an X-ray powder diffraction pattern as shown in Figure 2, the X-ray powder diffraction pattern being obtained by a powder diffractometer at CuKα radiation.

2. A process for preparation of ivabradine hydrochloride form X according to claim 1, which is **characterized in that** it comprises the step of drying ivabradine hydrochloride form Z for at least 2 hours at 20-30°C, ivabradine hydrochloride form Z being **characterized by** an X-ray powder diffraction pattern having peaks at 3.9, 15.1, 16.2, 16.6 and 17.8 ±0.2 degrees two-theta, the X-ray powder diffraction pattern being obtained by a powder diffractometer at CuKα radiation.

3. A process for preparation of ivabradine hydrochloride form X according to claim 2, which is **characterized in that** ivabradine hydrochloride form Z is prepared by a process for preparation of ivabradine hydrochloride form Z, which process comprises the steps of:
a) dissolving amorphous ivabradine hydrochloride in isopropanol at 15-30°C;
b) stirring the obtained solution at 15-30°C until ivabradine hydrochloride starts to precipitate;
c) recovering said precipitate,
ivabradine hydrochloride form Z being **characterized by** an X-ray powder diffraction pattern having peaks at 3.9, 15.1, 16.2, 16.6 and 17.8 ±0.2 degrees two-theta, the X-ray powder diffraction pattern being obtained by a powder diffractometer at CuKα radiation.

4. A process for preparation of ivabradine hydrochloride form X according to claim 2, which is **characterized in that** ivabradine hydrochloride form Z is prepared by a process for preparation of ivabradine hydrochloride form Z, which process comprises the steps of:
a) dissolving ivabradine in isopropanol at 15-30°C;
b) addition of hydrochloric acid in isopropanol at 15-• 30°C;
c) stirring the obtained solution at 15-30°C until ivabradine hydrochloride starts to precipitate;
d) recovering said precipitate,
ivabradine hydrochloride form Z being **characterized by** an X-ray powder diffraction pattern having peaks at 3.9, 15.1, 16.2, 16.6 and 17.8 ±0.2 degrees two-theta, the X-ray powder diffraction pattern being obtained by a powder diffractometer at CuKα radiation.

5. Use of ivabradine hydrochloride form X according to claim 1 for the preparation of pharmaceutical composition.

6. Use of ivabradine hydrochloride form X according to claim 1 for the preparation of ivabradine hydrochloride form K, wherein the process for the preparation of ivabradine hydrochloride form K comprises the step of drying ivabradine hydrochloride form X for at least 2 hours at 50-80°C, ivabradine hydrochloride form K being **characterized by** an X-ray powder diffraction pattern as shown in Figure 3, the X-ray powder diffraction pattern being obtained by a powder diffractometer at **CuKα** radiation.

7. Ivabradine hydrochloride form X according to claim 1 for use as a medicament.

## Patentansprüche

1. Ivabradin-Hydrochlorid Form X, welche **gekennzeichnet ist durch** ein Röntgen-Pulverbeugungsmuster wie in Figur 2 gezeigt, wobei das Röntgen-Pulverbeugungsmuster durch ein Pulverdiffraktometer bei CuKα-Strahlung erhalten wird.

2. Verfahren zur Herstellung von Ivabradin-Hydrochlorid Form X gemäß Anspruch 1, welches **dadurch gekennzeichnet ist, dass** es den Schritt von Trocknen von Ivabradin-Hydrochlorid Form Z mindestens 2 Stunden lang bei 20-30°C umfasst, wobei Ivabradin-Hydrochlorid Form Z durch ein Röntgen-Pulverbeugungsmuster mit Peaks bei 3,9, 15,1, 16,2, 16,6 und 17,8 ± 0,2 Grad Zwei-Theta gekennzeichnet ist, wobei das Röntgen-Pulverbeugungsmuster durch ein Pulverdiffraktometer bei CuKα-Strahlung erhalten wird.

3. Verfahren zur Herstellung von Ivabradin-Hydrochlorid Form X gemäß Anspruch 2, welches **dadurch gekennzeichnet ist, dass** Ivabradin-Hydrochlorid Form Z durch ein Verfahren zur Herstellung von Ivabradin-Hydrochlorid Form Z hergestellt wird, wobei dieses Verfahren die folgenden Schritte umfasst:
a) Lösen von amorphem Ivabradin-Hydrochlorid in Isopropanol bei 15-30°C;
b) Rühren der erhaltenen Lösung bei 15-30°C bis zum Beginn einer Präzipitation von Ivabradin-Hydrochlorid;
c) Gewinnen des Präzipitats,
wobei Ivabradin-Hydrochlorid Form Z durch ein Röntgen-Pulverbeugungsmuster mit Peaks bei 3,9, 15,1, 16,2, 16,6 und 17,8 ± 0,2 Grad Zwei-Theta gekennzeichnet ist, wobei das Röntgen-Pulverbeugungsmuster durch ein Pulverdiffraktometer bei CuKα-Strahlung erhalten wird.

4. Verfahren zur Herstellung von Ivabradin-Hydrochlorid Form X gemäß Anspruch 2, welches **dadurch gekennzeichnet ist, dass** Ivabradin-Hydrochlorid Form Z durch ein Verfahren zur Herstellung von Ivabradin-Hydrochlorid Form Z hergestellt wird, wobei dieses Verfahren die folgenden Schritte umfasst:
a) Lösen von Ivabradin in Isopropanol bei 15-30°C;
b) Zugeben von Chlorwasserstoffsäure in Isopropanol bei 15-30°C;
c) Rühren der erhaltenen Lösung bei 15-30°C bis zum Beginn einer Präzipitation von Ivabradin-Hydrochlorid;
d) Gewinnen des Präzipitats,
wobei Ivabradin-Hydrochlorid Form Z durch ein Röntgen-Pulverbeugungsmuster mit Peaks bei 3,9, 15,1, 16,2, 16,6 und 17,8 ± 0,2 Grad Zwei-Theta gekennzeichnet ist, wobei das Röntgen-Pulverbeugungsmuster durch ein Pulverdiffraktometer bei CuKα-Strahlung erhalten wird.

5. Verwendung von Ivabradin-Hydrochlorid Form X gemäß Anspruch 1 zur Herstellung einer pharmazeutischen Zusammensetzung.

6. Verwendung von Ivabradin-Hydrochlorid Form X gemäß Anspruch 1 zur Herstellung von Ivabradin-Hydrochlorid Form K, wobei das Verfahren zur Herstellung von Ivabradin-Hydrochlorid Form K den Schritt von Trocknen von Ivabradin-Hydrochlorid Form X mindestens 2 Stunden lang bei 50-80°C umfasst, wobei Ivabradin-Hydrochlorid Form K **gekennzeichnet ist durch** ein Röntgen-Pulverbeugungsmuster wie in Figur 3 gezeigt, wobei das Röntgen-Pulverbeugungsmuster durch ein Pulverdiffraktometer bei CuKα-Strahlung erhalten wird.

7. Ivabradin-Hydrochlorid Form X gemäß Anspruch 1 zur Verwendung als ein Medikament.

## Revendications

1. Chlorhydrate d'ivabradine de forme X qui est **caractérisée par** un spectre de diffraction des rayons X par la poudre, tel qu'il est illustré dans la Figure 2, le spectre de diffraction des rayons X par la poudre étant obtenu par un diffractomètre de poudre à un rayonnement CuKα.

2. Procédé de préparation de chlorhydrate d'ivabradine de forme X selon la revendication 1, qui est **caractérisé en ce qu'**il comprend l'étape de séchage de la forme Z de chlorhydrate d'ivabradine pendant au moins 2 heures à 20-30° C, la forme Z de chlorhydrate d'ivabradine étant **caractérisée par** un spectre de diffraction des rayons X par la poudre présentant des pics à 3,9, 15,1, 16,2, 16,6 et 17,8 +/- 0,2 degré 2 Thêta, le spectre de diffraction des rayons X par la poudre étant obtenu par un diffractomètre de poudre à un rayonnement CuKα.

3. Procédé de préparation de chlorhydrate d'ivabradine de forme X selon la revendication 2, **caractérisé en ce que** la forme Z de chlorhydrate d'ivabradine est préparée par un procédé de préparation de forme Z de chlorhydrate d'ivabradine, lequel procédé comprend les étapes suivantes:
a) dissolution de chlorhydrate d'ivabradine amorphe dans de l'isopropanol à 15-30°C ;
b) agitation de la solution obtenue à 15-30°C jusqu'à ce que le chlorhydrate d'ivabradine commence à précipiter ;
c) récupération dudit précipité,
la forme Z de chlorhydrate d'ivabradine étant **caractérisée par** un spectre de diffraction des rayons X par la poudre présentant des pics à 3,9, 15,1, 16,2, 16,6 et 17,8 + /- 0,2 degré 2 Thêta, le spectre de diffraction des rayons X par la poudre étant obtenu par un diffractomètre de poudre à un rayonnement CuKa.

4. Procédé de préparation de chlorhydrate d'ivabradine de forme X selon la revendication 2, **caractérisé en ce que** la forme Z de chlorhydrate d'ivabradine est préparée par un procédé de préparation de forme Z de chlorhydrate d'ivabradine, lequel procédé comprend les étapes suivantes:
a) dissolution d'ivrabradine dans de l'isopropanol à 15-30°C ;
b) addition d'acide chlorhydrique dans l'isopropanol à 15-30°C ;
b) agitation de la solution obtenue à 15-30°C jusqu'à ce que le chlorhydrate d'ivabradine commence à précipiter ;
c) récupération dudit précipité,
la forme Z de chlorhydrate d'ivabradine étant **caractérisée par** un spectre de diffraction des rayons X par la poudre présentant des pics à 3,9, 15,1, 16,2, 16,6 et 17,8 + /- 0,2 degré 2 Thêta, le spectre de diffraction des rayons X par la poudre étant obtenu par un diffractomètre de poudre à un rayonnement CuKa.

5. Utilisation de chlorhydrate d'ivabradine de forme X selon la revendication 1 pour la préparation d'une composition pharmaceutique.

6. Utilisation de chlorhydrate d'ivabradine de forme X selon la revendication 1 pour la préparation de chlorhydrate d'ivabradine de forme K, dans laquelle le procédé de préparation de chlorhydrate d'ivabradine de forme K comprend l'étape de séchage du chlorhydrate d'ivabradine de forme X pendant au moins 2 heures à 50-80°C, le chlorhydrate d'ivabradine de forme K étant **caractérisé par** un spectre de diffraction des rayons X par la poudre, tel qu'il est illustré dans la Figure 3, le spectre de diffraction des rayons X par la poudre étant obtenu par un diffractomètre de poudre à un rayonnement CuKa.

7. Chlorhydrate d'ivabradine de forme X selon la revendication 1, à utiliser comme médicament.
